# EUROPEAN PATENT APPLICATION

(11) **EP 3 492 453 A1**
(43) Date of publication of application: **05.06.2019**
(21) Application number: 17834377.8
(22) Date of filing: 26.07.2017
(51) Int. Cl.: C07D 231/14

(54) **METHOD OF PRODUCING FLUORINE-CONTAINING PYRAZOLE CARBOXYLIC ACID HALIDE**

(30) Priority: 29.07.2016 JP 2016150521
(71) Applicant: AGC INC., Chiyoda-ku, Tokyo 1008405 (JP)
(72) Inventor: ISHIBASHI, Yuichiro, Tokyo 100-8405 (JP); MATSUMURA, Yasushi, Tokyo 100-8405 (JP); YOKOKOJI, Osamu, Tokyo 100-8405 (JP); SHIMIZU, Tamaki, Tokyo 100-8405 (JP)
(74) Representative: J A Kemp
(86) International application number: PCT/JP2017/026963
(87) International publication number: WO 2018/021375

(57) **Abstract**

The present invention provides a method capable of more simply and efficiently producing fluorine-containing pyrazolecarboxylic acid halides useful as pharmaceutical or agrochemical intermediates.

The present invention relates to a method of producing a compound represented by the formula (a), which comprises reacting a compound represented by the formula (c) with a halogenating agent in a non-aqueous system; 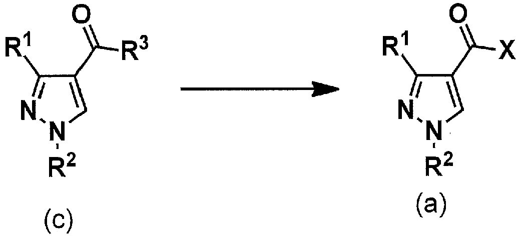 wherein
R¹ is a fluoroalkyl group having 1 to 3 carbon atoms,
R² is a hydrogen atom or an alkyl group having 1 to 6 carbon atoms,
R³ is an alkyl group having 1 to 6 carbon atoms, and
X is a halogen atom.

## Description

### Technical Field

The present invention relates to a method of producing fluorine-containing pyrazolecarboxylic acid halides useful as pharmaceutical or agrochemical intermediates.

### Background Art

Fluorine-containing pyrazolecarboxylic acid halides such as 3-difluoromethyl-1-methyl-1H-pyrazole-4-carbonyl chloride and the like are intermediates useful for pyrazolylcarboxanilide fungicides (see, for example, Patent Documents 1 and 2).

Moreover, Patent Document 3 discloses a method of obtaining a compound represented by the following formula (b¹) by reacting a compound represented by the following formula (c¹) with sodium hypochlorite in an aqueous system.

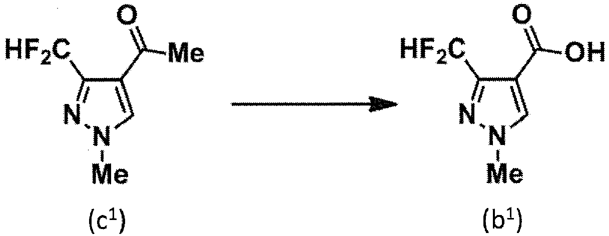

### Document List

### Patent Document

Patent Document 1: WO 03/070705
Patent Document 2: WO 03/074491
Patent Document 3: CN 105541716 specification

### Summary of the Invention

### Problems to be Solved by the Invention

A method of reacting a compound represented by the formula (b¹), which is obtained according to the method described in Patent Document 3, with phosgene or thionyl chloride may be conceived as a method of producing fluorine-containing pyrazolecarboxylic acid halides as typified by a compound represented by the following formula (a¹) (3-difluoromethyl-1-methyl-1H-pyrazole-4-carbonyl chloride), as shown in the following scheme.

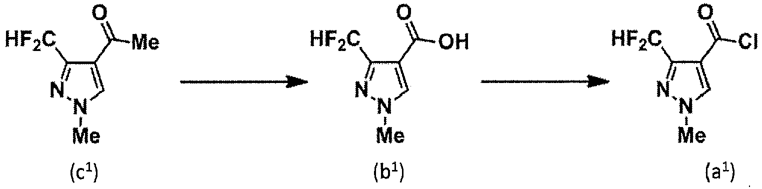

However, this scheme needs two-step reaction, and therefore, more simple production method is desired from the industrial aspect.

Fluorine-containing pyrazolylalkylketones as typified by a compound represented by the formula (c¹), i.e., pyrazole derivatives wherein the 1-position is substituted with an alkyl group, the 3-position is substituted with a fluoroalkyl group, and the 4-position is substituted with an alkylketone group (alkylcarbonyl group), seem to have a unique property. However, the details have not been yet known.

The aim of the present invention is to provide a method capable of more simply and efficiently producing fluorine-containing pyrazolecarboxylic acid halides useful as pharmaceutical or agrochemical intermediates.

### Means of Solving the Problems

The present inventors have conducted intensive studies on the reaction properties of fluorine-containing pyrazolylalkylketones, and have found that fluorine-containing pyrazolecarboxylic acid halides can be obtained by reacting fluorine-containing pyrazolylalkylketones with a halogenating agent in a non-aqueous system. That is, fluorine-containing pyrazolecarboxylic acid halides can be obtained from fluorine-containing pyrazolylalkylketones by one-step reaction.

Accordingly, the present invention comprises the following inventions.

[1] A method of producing a compound represented by the formula (a), which comprises reacting a compound represented by the formula (c) with a halogenating agent in a non-aqueous system; 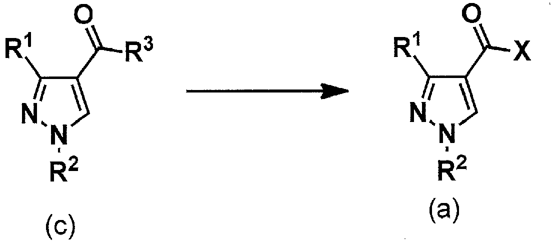 wherein
   R¹ is a fluoroalkyl group having 1 to 3 carbon atoms,
   R² is a hydrogen atom or an alkyl group having 1 to 6 carbon atoms,
   R³ is an alkyl group having 1 to 6 carbon atoms, and
   X is a halogen atom.
[2] The method according to the above-mentioned [1], wherein R¹ is a monofluoromethyl group, a difluoromethyl group or a trifluoromethyl group, R² is a hydrogen atom, a methyl group, an ethyl group, an n-propyl group or an isopropyl group, and R³ is a methyl group, an ethyl group, an n-propyl group or an isopropyl group.
[3] The method according to the above-mentioned [1] or [2], wherein the halogenating agent is a chlorinating agent, and X is a chlorine atom.
[4] The method according to the above-mentioned [3], wherein the chlorinating agent is chlorine (Cl₂), p-toluenesulfonyl chloride, methanesulfonyl chloride, oxalyl chloride, phosgene, sulfur dichloride, sulfur monochloride, thionyl chloride, sulfuryl chloride or phosphorus pentachloride.
[5] The method according to any one of the above-mentioned [1] to [4], wherein the reaction is carried out in the presence of a non-aqueous solvent.
[6] The method according to the above-mentioned [5], wherein the non-aqueous solvent is an aromatic halide or an aliphatic halide.

### Effect of the Invention

According to the production method of the present invention, fluorine-containing pyrazolecarboxylic acid halides useful as pharmaceutical or agrochemical intermediates can be produced efficiently on industrial scale.

### Description of Embodiments

In the present specification, a compound represented by the formula (x) may also be referred to as compound (x). In the present specification, numerical range shown by using "to" means the range including the numerical values described before and after "to" as minimum and maximum values.

The embodiments of the present invention are explained below in detail.

The present invention provides a method of producing a compound represented by the following formula (a) (hereinafter, also simply referred to as "compound (a)"), i.e., a fluorine-containing pyrazolecarboxylic acid halide, which comprises reacting a compound represented by the following formula (c) (hereinafter, also simply referred to as "compound (c)") with a halogenating agent in a non-aqueous system. 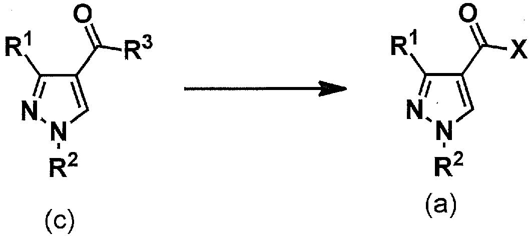 In the formula, R¹ is a fluoroalkyl group having 1 to 3 carbon atoms.

The fluoroalkyl group means a group wherein one or more hydrogen atoms of the alkyl group are replaced with fluorine atoms.

R¹ is preferably a monofluoromethyl group, a difluoromethyl group or a trifluoromethyl group, particularly preferably a difluoromethyl group or a trifluoromethyl group.

In the formula, R² is a hydrogen atom or an alkyl group having 1 to 6 carbon atoms.

R² is preferably a hydrogen atom, a methyl group, an ethyl group, an n-propyl group or an isopropyl group, particularly preferably a methyl group.

In the formula, R³ is an alkyl group having 1 to 6 carbon atoms.

R³ is preferably a methyl group, an ethyl group, an n-propyl group or an isopropyl group, particularly preferably a methyl group.

In the formula, X is a halogen atom. Examples of the halogen atom include a fluorine atom, a chlorine atom, a bromine atom and an iodine atom.

X is preferably a chlorine atom or a bromine atom, particularly preferably a chlorine atom.

As a preferable embodiment, the compound wherein R¹ is a monofluoromethyl group, a difluoromethyl group or a trifluoromethyl group, R² is a hydrogen atom, a methyl group, an ethyl group, an n-propyl group or an isopropyl group, and R³ is a methyl group, an ethyl group, an n-propyl group or an isopropyl group is preferable. In this embodiment, R³ is particularly preferably a methyl group.

Compound (c) can be obtained according to a known method (for example, the method described in Patent Document 3).

Alternatively, compound (c) can be also obtained from a compound represented by the following formula (c'). Compound (c') corresponds to compound (c) protected by an acetal.

In the formula, n is an integer of 1 to 4.

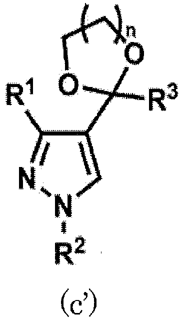

When compound (c') is used, then compound (c') is, for example, preferably chemically converted to compound (c) under acidic condition (deprotection). The obtained compound (c) can be subjected to the production method of the present invention after isolation or sequentially without isolation.

Specific examples of compound (c) include the following compounds.

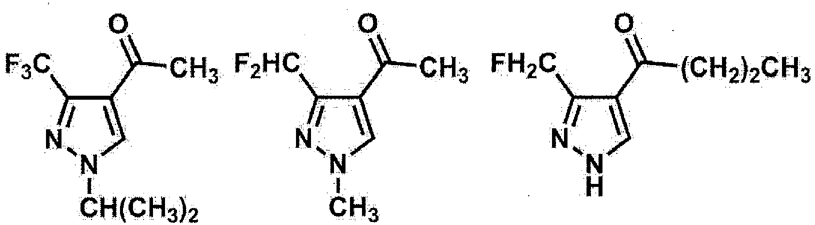

Specific examples of compound (a) include the following compounds. In the formulas, X is a halogen atom.

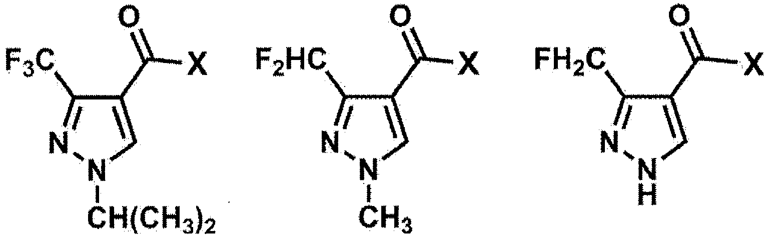

The kind of the halogenating agent is not particularly limited, and examples thereof include conventional halogenating agents (e.g., a fluorinating agent, a chlorinating agent, a brominating agent, an iodinating agent). Among them, a chlorinating agent and a brominating agent are preferable, and a chlorinating agent is particularly preferable. In the production method of the present invention, when the halogenating agent is a chlorinating agent, then compound (a) wherein X is a chlorine atom is obtained, and when the halogenating agent is a brominating agent, then compound (a) wherein X is a bromine atom is obtained.

The chlorinating agent is preferably chlorine (Cl₂), p-toluenesulfonyl chloride (tosyl chloride), methanesulfonyl chloride (mesyl chloride), oxalyl chloride, phosgene, sulfur dichloride, sulfur monochloride, thionyl chloride, sulfuryl chloride or phosphorus pentachloride. It is particularly preferably sulfur monochloride, thionyl chloride or sulfuryl chloride, from the aspect of easy handling, economic efficiency and reaction work-up (isolation and purification of compound (a)).

The brominating agent is preferably bromine (Br₂), trichlorobromomethane, tetrabromomethane, phosphorus tribromide or boron tribromide.

The amount of the halogenating agent to be used is preferably 1 equivalent or more relative to compound (c), from the aspect of conversion and selectivity of the reaction, preferably 10 equivalent or less, particularly preferably 6 equivalent or less, relative to compound (c), from the aspect of inhibition of side reaction. The above-mentioned equivalent means molar equivalent.

For example, when sulfur monochloride is used as a halogenating agent, it is preferably used in an amount of 3 to 6 mol, per 1 mol of compound (c).

The halogenating agent may be used alone or in combination of two or more kinds thereof.

Moreover, the production method of the present invention is preferably carried out in the presence of a basic compound, from the aspect of yield of compound (a).

The basic compound is preferably a basic compound having only proton affinity, but not having acidic property, specifically preferably a nitrogen-containing compound (an amine compound), particularly preferably a nitrogen-containing aromatic compound. Specific examples of the basic compound include pyridine, pyrimidine, pyridazine, pyrazine, oxazole, thiazole, quinoline and the like.

The amount of the basic compound to be used is preferably exceeding 0 to 0.25 mol, particularly preferably 0.01 to 0.15 mol, per 1 mol of compound (c).

The production method of the present invention is carried out in a non-aqueous system, from the aspect of inhibition of hydrolysis of the produced compound (a). The non-aqueous system herein means a system wherein water is substantively absent, specifically, a system wherein its moisture content is maintained at 1000 mass ppm or less. The moisture content in the system is represented by mass ratio of water relative to all materials placed in the system. The above-mentioned moisture content is measured by Karl Fischer moisture measurement method.

The production method of the present invention is preferably carried out in the presence of a non-aqueous solvent. The non-aqueous solvent means a solvent other than water. The moisture content of the non-aqueous solvent (mass ratio of water relative to the non-aqueous solvent) is preferably 1000 mass ppm or less.

The non-aqueous solvent is preferably a solvent inert to the halogenating agent, and specific examples thereof include ether solvents such as diethyl ether, tetrahydrofuran, dioxane, 1,2-dimethoxyethane and the like; aromatic hydrocarbon solvents such as benzene, toluene, xylene, mesitylene and the like; aromatic halides such as monochlorobenzene, dichlorobenzene and the like; aliphatic hydrocarbon solvents such as hexane, heptane, octane, cyclohexane and the like; and aliphatic halides such as methylene chloride, chloroform, carbon tetrachloride, 1,2-dichloroethane and the like.

The non-aqueous solvent is preferably an aromatic halide or an aliphatic halide, particularly preferably monochlorobenzene, dichloromethane or chloroform.

The amount of the non-aqueous solvent to be used is preferably 1 to 50-fold amount (mass standard), particularly preferably 2 to 15-fold amount (mass standard), relative to compound (c).

In the production method of the present invention, the procedure of mixing compound (c) and a halogenating agent is not particularly limited.

For example, a procedure of adding a halogenating agent to compound (c) or a solution containing compound (c) and a non-aqueous solvent can be employed. The halogenating agent may be added in the form of a solution in a non-aqueous solvent.

Alternatively, a procedure of adding compound (c) to a halogenating agent or a solution containing a halogenating agent and a non-aqueous solvent can also be employed. Compound (c) may be added in the form of a solution in a non-aqueous solvent.

A method of mixing compound (c) and a halogenating agent may be a method of mixing both in batch (batch mixing method), or a method of mixing compound (c) or a halogenating agent by small portions into the other (portioning mixing method).

In the portioning mixing method, the number of portions to be mixed into the other is not particularly limited as long as it is two or more, and the upper limit is usually five.

Specific examples of the portioning mixing method include a method of dividing compound (c) or a solution containing compound (c) and a non-aqueous solvent into two or more portions, and mixing these portions into a halogenating agent.

In mixing of compound (c) and a halogenating agent, the one may be added to the other under stirring, if necessary.

In the production method of the present invention, the reaction temperature is preferably 0 to 250°C, particularly preferably 0 to 200°C, from the aspect of efficient reaction progress due to inhibition of side reaction. The reaction temperature may be varied during the reaction, if necessary.

The reaction atmosphere is not particularly limited, and it is preferably an inert gas (nitrogen etc.) atmosphere, in order to maintain the conditions of the non-aqueous system, as mentioned above.

The pressure of the reaction atmosphere may be increased pressure, reduced pressure or atmospheric pressure, preferably atmospheric pressure.

In the production method of the present invention, the method of isolating compound (a) from the reaction system after the completion of the reaction is not particularly limited. For example, compound (a) with high purity can be easily isolated by solvent extraction or crystallization.

Compound (a) obtained by the production method of the present invention is useful as intermediates for antimicrobials, fungicides, insecticides, or bulk pharmaceuticals or agrochemicals, for example, useful as intermediates for the following antimicrobials, fungicides, or bulk pharmaceuticals or agrochemicals.

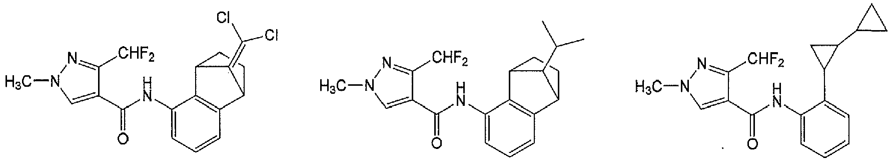

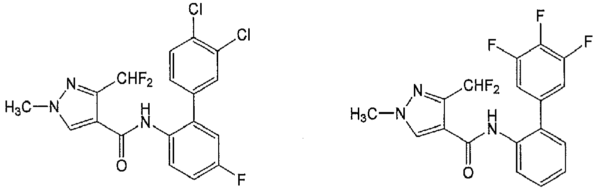

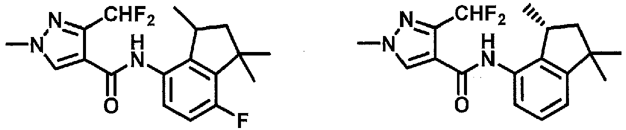

As a method of producing antimicrobials, fungicides, insecticides, or bulk pharmaceuticals or agrochemicals using compound (a), a method of reacting compound (a) with each of amines represented by the following formulas can be employed. Each reaction can be carried out according to a known method.

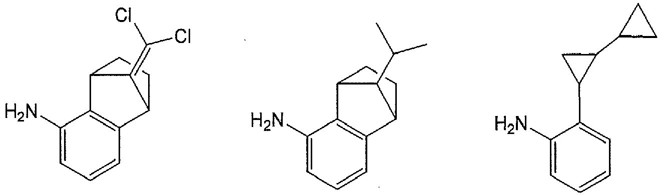

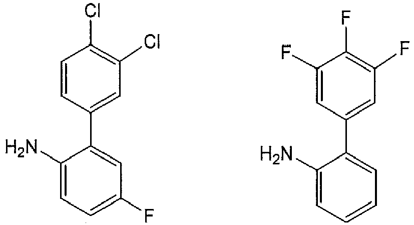

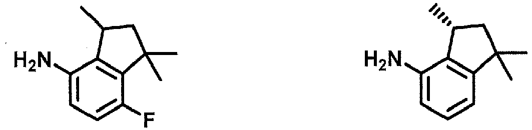

According to the production method of the present invention, the number of steps is reduced, and compound (a) can be efficiently produced, compared to conventional art. That is, chloroalkane (chloroform, etc.) generated as a by-product in an oxidation reaction using sodium hypochlorite, as known in the conventional art, is not substantially generated in the production method of the present invention. Therefore, according to the production method of the present invention, antimicrobials, fungicides, or bulk pharmaceuticals or agrochemicals can be efficiently produced compared to conventional art.

### Examples

The present invention is explained in the following by referring to Example, which are not to be construed as limitative. NMR apparatus used for analysis in Example is JNM-ECP400 (400MHz) manufactured by JEOL Ltd. The unit of yield (%) described in Example is mol %.

### [Example 1] Synthesis of compound (a¹)

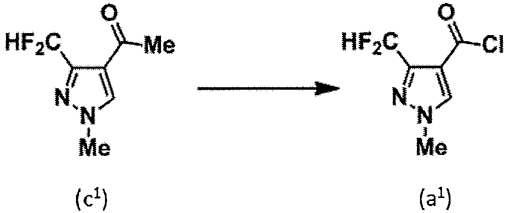

Sulfur monochloride (5.40 g, 40 mmol) was placed in a reactor. The reactor inside was kept under nitrogen atmosphere, and the system in the below-mentioned reaction was also kept in a non-aqueous state (moisture content: 1000 mass ppm or less).

First, a non-aqueous solution containing compound (c¹) (1.74 g, 10 mmol), monochlorobenzene (13.4 g) and pyridine (0.12 g, 1.5 mmol) was prepared.

Next, 1/5 volume of the non-aqueous solution was placed in the reactor while the solution in the reactor was stirred, and the solution in the reactor was heated at 80 to 90°C for 10 min, and then cooled to 25°C. Then, the residual non-aqueous solution (4/5 volume) was placed in the reactor, and the obtained solution in the reactor was stirred at 25°C for 22 hr. Then, the solution was stirred at 137°C. for 27 hr. The obtained solution in the reactor was analyzed by ¹⁹F-NMR (internal standard material: 3,4,5-trichlorobenzotrifluoride), and thereby, the production of compound (a¹) was confirmed. The yield of compound (a¹) was 88% on the basis of the used amount of compound (c¹).

### Industrial Applicability

As is clear from the above-mentioned results, according to the production method of the present invention, compound (a), i.e., a fluorine-containing pyrazolecarboxylic acid halide, can be obtained in a high-yield of 80% or more, from compound (c), i.e., a fluorine-containing pyrazolylalkylketone, by one-step reaction.

## Claims

1. A method of producing a compound represented by the formula (a), which comprises reacting a compound represented by the formula (c) with a halogenating agent in a non-aqueous system; 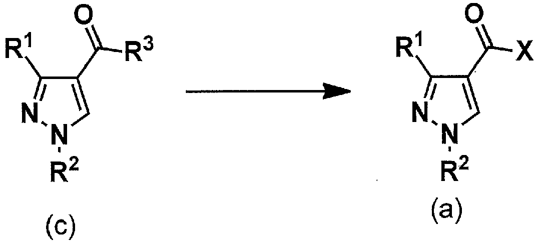 wherein
R¹ is a fluoroalkyl group having 1 to 3 carbon atoms,
R² is a hydrogen atom or an alkyl group having 1 to 6 carbon atoms,
R³ is an alkyl group having 1 to 6 carbon atoms, and
X is a halogen atom.

2. The method according to claim 1, wherein R¹ is a monofluoromethyl group, a difluoromethyl group or a trifluoromethyl group, R² is a hydrogen atom, a methyl group, an ethyl group, an n-propyl group or an isopropyl group, and R³ is a methyl group, an ethyl group, an n-propyl group or an isopropyl group.

3. The method according to claim 1 or 2, wherein the halogenating agent is a chlorinating agent, and X is a chlorine atom.

4. The method according to claim 3, wherein the chlorinating agent is chlorine (Cl₂), p-toluenesulfonyl chloride, methanesulfonyl chloride, oxalyl chloride, phosgene, sulfur dichloride, sulfur monochloride, thionyl chloride, sulfuryl chloride or phosphorus pentachloride.

5. The method according to any one of claims 1 to 4, wherein the reaction is carried out in the presence of a non-aqueous solvent.

6. The method according to claim 5, wherein the non-aqueous solvent is an aromatic halide or an aliphatic halide.
